# EUROPEAN PATENT APPLICATION

(11) **EP 4 159 239 A1**
(43) Date of publication of application: **05.04.2023**
(21) Application number: 21812951.8
(22) Date of filing: 24.05.2021
(51) Int. Cl.: A61K 45/00, A61P 21/00, A61P 25/00, A61P 25/02, A61P 25/16, A61P 25/28, A61P 43/00, A61K 31/445, A61K 31/496, A61K 31/5375

(54) **AGENT FOR PROTECTING AND/OR REGENERATING NEUROMUSCULAR JUNCTIONS**

(30) Priority: 25.05.2020 JP 2020090194
(71) Applicant: ONO Pharmaceutical Co., Ltd., Osaka-shi, Osaka 541-8526 (JP)
(72) Inventor: NAKANISHI, Masaya, Mishima-gun, Osaka 618-8585 (JP); KANAYA, Kazuo, Mishima-gun, Osaka 618-8585 (JP); KISA, Fumihiko, Mishima-gun, Osaka 618-8585 (JP); ITO, Ryo, Mishima-gun, Osaka 618-8585 (JP); WATANABE, Ryosuke, Mishima-gun, Osaka 618-8585 (JP)
(74) Representative: J A Kemp LLP
(86) International application number: PCT/JP2021/019622
(87) International publication number: WO 2021/241504

(57) **Abstract**

In one aspect, the present invention provides an agent for protecting and/or regenerating a neuromuscular junction or an agent for treating and/or preventing a disease associated with a disorder of a neuromuscular junction, the agent containing an FF-MAS metabolism inhibitor. In one aspect, the present invention also provides a method for protecting and/or regenerating a neuromuscular junction or a method for treating and/or preventing a disease associated with a disorder of a neuromuscular junction, the method including administering an effective amount of an FF-MAS metabolism inhibitor to a mammal. In one aspect, the present invention also provides an FF-MAS metabolism inhibitor for use in protecting and/or regenerating a neuromuscular junction or for use in treating and/or preventing a disease associated with a disorder of a neuromuscular junction. In one aspect, the present invention also provides use of an FF-MAS metabolism inhibitor for manufacturing an agent for protecting and/or regenerating a neuromuscular junction or for manufacturing an agent for treating and/or preventing a disease associated with a disorder of a neuromuscular junction.

## Description

### TECHNICAL FIELD

The present invention relates to a protection use of a neuromuscular junction and/or a nervous system cell by a follicular fluid meiosis-activating sterol (FF-MAS) metabolism inhibitor, and the like.

### BACKGROUND ART

The nervous system is roughly divided into a central nervous system and a peripheral nervous system, and is composed of nerve cells and glial cells such as Schwann cells, oligodendrocytes, and microglia. Furthermore, in the peripheral nervous system, a motor neuron forms a neuromuscular junction together with a muscle tissue, and acetylcholine released from the motor neuron terminal stimulates acetylcholine receptors present in muscle cells to cause muscle contraction. Amyotrophic lateral sclerosis is a disease in which muscle functions are declined by degeneration of motor neurons and neuromuscular junctions, and although there is a treatment method for the purpose of ameliorating symptoms, suppressing progression, or the like, the effect thereof is not necessarily sufficient, and development of a new therapeutic agent is desired (Non Patent Literature 1). On the other hand, in the same disease, it has been reported that a disorder of a neuromuscular junction occurs from an early stage of onset, and a therapeutic effect by ameliorating the disorder can be expected (Non Patent Literatures 2 and 3).

Meanwhile, Patent Literature 1 and Non Patent Literatures 4 and 5 report that among sterol metabolic enzymes, inhibitors of cytochrome P450 family 51 (CYP51), sterol C 14-reductase, or emopamil binding protein (EBP) have an oligodendrocyte differentiation-promoting action.

### CITATIONS LIST

### Patent Literature

Patent Literature 1: WO 2018/022904 A

### Non Patent Literatures

Non Patent Literature 1: Practical Guideline for Amyotrophic Lateral Sclerosis (ALS) 2013
Non Patent Literature 2: EMBO Molecular Medicine, Vol. 9, (2017) 880-889
Non Patent Literature 3: Experimental Neurology, 185 (2004) 232-240
Non Patent Literature 4: Nature, Vol. 560 (2018), 372-376
Non Patent Literature 5: Cell Chemical Biology 26, (2019) 593-599

### SUMMARY OF INVENTION

### TECHNICAL PROBLEMS

An object of the present invention is to provide a medicine for protecting and/or regenerating a neuromuscular junction, or an agent for protecting and/or regenerating a nervous system cell.

### SOLUTIONS TO PROBLEMS

As a result of intensive studies to achieve the above object, the present inventors have found that an FF-MAS metabolism inhibitor, a sterol C14-reductase inhibitor, or a transmembrane 7 superfamily member 2 (TM7SF2) inhibitor (hereinafter, it may be referred to as an FF-MAS metabolism inhibitor or the like) has a protecting and/or regenerating action on a neuromuscular junction, and further has a protecting and/or regenerating action on a nervous system cell.

The present invention has, for example, the following aspects.
[1] An agent for protecting and/or regenerating a neuromuscular junction or an agent for protecting and/or regenerating a nervous system cell, the agent containing a metabolism inhibitor of sterol constituting a cholesterol synthesis pathway.
[2] The agent according to [1], wherein the metabolism inhibitor of sterol constituting a cholesterol synthesis pathway is an FF-MAS metabolism inhibitor.
[3] The agent according to [1] or [2], for protecting and/or regenerating a neuromuscular junction.
[4] The agent according to [1] or [2], for protecting and/or regenerating a nervous system cell.
[5] The agent according to [4], wherein the protection and/or regeneration of a nervous system cell is mediated by a neurite outgrowth action.
[6] The agent according to [4], wherein the protection and/or regeneration of a nervous system cell is mediated by a Schwann cell differentiation-promoting action.
[7] An agent for protecting and/or regenerating a neuromuscular junction, the agent containing an FF-MAS metabolism inhibitor (for example, a sterol C14-reductase inhibitor or a TM7SF2 inhibitor).
[8] A neurite outgrowth agent containing an FF-MAS metabolism inhibitor (for example, a sterol C14-reductase inhibitor or a TM7SF2 inhibitor).
[9] A Schwann cell differentiation-promoting agent containing an FF-MAS metabolism inhibitor (for example, a sterol C14-reductase inhibitor or a TM7SF2 inhibitor).
[10] The agent according to any one of [2] to [9], wherein the FF-MAS metabolism inhibitor is a sterol C14-reductase inhibitor.
[11] The agent according to [10], wherein the sterol C14-reductase inhibitor is a TM7SF2 inhibitor and/or an LBR inhibitor.
[12] The agent according to [10], wherein the sterol C14-reductase inhibitor is a TM7SF2 inhibitor (preferably a selective TM7SF2 inhibitor).
[13] The agent according to [10], wherein the sterol C14-reductase inhibitor is an LBR inhibitor (preferably a selective LBR inhibitor).
[14] The agent according to [10], wherein the sterol C14-reductase inhibitor is a TM7SF2 and LBR dual inhibitor (preferably a selective TM7SF2 and LBR dual inhibitor).
[15] The agent according to [10], wherein the sterol C14-reductase inhibitor is a substance that selectively inhibits sterol C14-reductase (for example, a selective TM7SF2 inhibitor, a selective LBR inhibitor, or a selective TM7SF2 and LBR dual inhibitor, preferably a selective TM7SF2 inhibitor).
[16] An agent for protecting and/or regenerating a neuromuscular junction or an agent for protecting and/or regenerating a nervous system cell, the agent containing FF-MAS or 14-dehydrozymosterol or a derivative thereof.
[17] An agent for protecting and/or regenerating a neuromuscular junction or an agent for protecting and/or regenerating a nervous system cell, the agent containing an agent having an action of increasing an FF-MAS concentration and/or a 14-dehydrozymosterol concentration.
[18] The agent according to any one of [1] to [17], for treating and/or preventing a neurodegenerative disease.
[19] The agent according to any one of [1] to [3], [7], or [10] to [17], for treating and/or preventing a disease associated with a disorder of a neuromuscular junction.
[20] The agent according to [19], wherein the disease associated with a disorder of a neuromuscular junction is a neurodegenerative disease or a myogenic disease.
[21] The agent according to [20], wherein the disease associated with a disorder of a neuromuscular junction is the neurodegenerative disease.
[22] The agent according to [18] or [21], wherein the neurodegenerative disease is peripheral neuropathy, Alzheimer's disease, Parkinson's disease, or amyotrophic lateral sclerosis.
[23] The agent according to [20], wherein the disease associated with a disorder of a neuromuscular junction is the myogenic disease.
[24] The agent according to [23], wherein the myogenic disease is myasthenia gravis, muscular dystrophy, sarcopenia, or myopathy.
[25] An agent for protecting and/or regenerating a nervous system cell, the agent containing an FF-MAS metabolism inhibitor (for example, a sterol C14-reductase inhibitor or a TM7SF2 inhibitor).
[26] The agent according to [25], wherein the protection and/or regeneration of a nervous system cell is mediated by a neurite outgrowth action.
[27] The agent according to [25], wherein the protection and/or regeneration of a nervous system cell is mediated by a Schwann cell differentiation-promoting action.
[28] An agent for protecting and/or regenerating a neuromuscular junction, the agent containing a TM7SF2 inhibitor (preferably a selective TM7SF2 inhibitor).
[29] The agent according to [28], for treating and/or preventing a neurodegenerative disease.
[30] The agent according to [28], for treating and/or preventing a disease associated with a disorder of a neuromuscular junction.
[31] The agent according to [30], wherein the disease associated with a disorder of a neuromuscular junction is a neurodegenerative disease or a myogenic disease.
[32] The agent according to [31], wherein the disease associated with a disorder of a neuromuscular junction is the neurodegenerative disease.
[33] The agent according to [29] or [32], wherein the neurodegenerative disease is peripheral neuropathy, Alzheimer's disease, Parkinson's disease, or amyotrophic lateral sclerosis.
[34] The agent according to [31], wherein the disease associated with a disorder of a neuromuscular junction is the myogenic disease.
[35] The agent according to [34], wherein the myogenic disease is myasthenia gravis, muscular dystrophy, sarcopenia, or myopathy.
[2-1] An agent for treating and/or preventing a neurodegenerative disease, the agent containing an FF-MAS metabolism inhibitor (for example, a sterol C14-reductase inhibitor or a TM7SF2 inhibitor).
[2-2] An agent for treating and/or preventing a myogenic disease, the agent containing an FF-MAS metabolism inhibitor (for example, a sterol C14-reductase inhibitor or a TM7SF2 inhibitor).
[2-3] An agent for treating and/or preventing a disease associated with a disorder of a neuromuscular junction, the agent containing an FF-MAS metabolism inhibitor (for example, a sterol C14-reductase inhibitor or a TM7SF2 inhibitor).
[2-4] The agent according to [2-3], for protecting and/or regenerating a neuromuscular junction.
[2-5] The agent according to [2-3] or [2-4], wherein the disease associated with a disorder of a neuromuscular junction is a neurodegenerative disease or a myogenic disease.
[2-6] The agent according to [2-5], wherein the disease associated with a disorder of a neuromuscular junction is a neurodegenerative disease.
[2-7] The agent according to [2-1] or [2-6], wherein the neurodegenerative disease is peripheral neuropathy, Alzheimer's disease, Parkinson's disease, or amyotrophic lateral sclerosis.
[2-8] The agent according to [2-6] or [2-7], further for protecting and/or regenerating a nervous system cell.
[2-9] The agent according to [2-8], wherein the protection and/or regeneration of a nervous system cell is neurite outgrowth.
[2-10] The agent according to [2-8], wherein the protection and/or regeneration of a nervous system cell is Schwann cell differentiation promotion.
[2-11] The agent according to [2-5], wherein the disease associated with a disorder of a neuromuscular junction is a myogenic disease.
[2-12] The agent according to [2-2] or [2-11], wherein the myogenic disease is myasthenia gravis, muscular dystrophy, sarcopenia, or myopathy.
[2-13] The agent according to any one of [2-1] to [2-12], wherein the FF-MAS metabolism inhibitor is a sterol C14-reductase inhibitor.
[2-14] The agent according to [2-13], wherein the sterol C14-reductase inhibitor is a TM7SF2 inhibitor and/or an LBR inhibitor.
[2-15] The agent according to [2-13], wherein the sterol C14-reductase inhibitor is a TM7SF2 inhibitor (preferably a selective TM7SF2 inhibitor).
[2-16] The agent according to [2-13], wherein the sterol C14-reductase inhibitor is an LBR inhibitor (preferably a selective LBR inhibitor).
[2-17] The agent according to [2-13], wherein the sterol C14-reductase inhibitor is a TM7SF2 and LBR dual inhibitor (preferably a selective TM7SF2 and LBR dual inhibitor).
[2-18] The agent according to [2-13], wherein the sterol C14-reductase inhibitor is a substance that selectively inhibits sterol C14-reductase (for example, a selective TM7SF2 inhibitor, a selective LBR inhibitor, or a selective TM7SF2 and LBR dual inhibitor, preferably a selective TM7SF2 inhibitor).
[2-19] An agent for treating and/or preventing a disease associated with a disorder of a neuromuscular junction, the agent containing FF-MAS or 14-dehydrozymosterol or a derivative thereof.
[2-20] An agent for treating and/or preventing a disease associated with a disorder of a neuromuscular junction, the agent containing an agent having an action of increasing an FF-MAS concentration and/or a 14-dehydrozymosterol concentration.
[2-21] An agent for treating and/or preventing a neurodegenerative disease, the agent containing a TM7SF2 inhibitor (preferably a selective TM7SF2 inhibitor).
[2-22] An agent for treating and/or preventing a myogenic disease, the agent containing a TM7SF2 inhibitor (preferably a selective TM7SF2 inhibitor).
[2-23] An agent for treating and/or preventing a disease associated with a disorder of a neuromuscular junction, the agent containing a TM7SF2 inhibitor (preferably a selective TM7SF2 inhibitor).
[2-24] The agent according to [2-23], for protecting and/or regenerating a neuromuscular junction.
[2-25] The agent according to [2-23] or [2-24], wherein the disease associated with a disorder of a neuromuscular junction is a neurodegenerative disease or a myogenic disease.
[2-26] The agent according to [2-25], wherein the disease associated with a disorder of a neuromuscular junction is a neurodegenerative disease.
[2-27] The agent according to [2-21] or [2-26], wherein the neurodegenerative disease is peripheral neuropathy, Alzheimer's disease, Parkinson's disease, or amyotrophic lateral sclerosis.
[2-28] The agent according to [2-26] or [2-27], further for protecting and/or regenerating a nervous system cell.
[2-29] The agent according to [2-28], wherein the protection and/or regeneration of a nervous system cell is neurite outgrowth.
[2-30] The agent according to [2-28], wherein the protection and/or regeneration of a nervous system cell is Schwann cell differentiation promotion.
[2-31] The agent according to [2-25], wherein the disease associated with a disorder of a neuromuscular junction is a myogenic disease.
[2-32] The agent according to [2-22] or [2-31], wherein the myogenic disease is myasthenia gravis, muscular dystrophy, sarcopenia, or myopathy.
[3-1] A method, using, as an index, a concentration of sterol (for example, FF-MAS, T-MAS, 14-dehydrozymosterol, or 14-dehydrozymostenol) constituting a cholesterol synthesis pathway in a biological sample of a patient with a neurodegenerative disease, for determining or predicting efficacy of an FF-MAS metabolism inhibitor (for example, a sterol C14-reductase inhibitor or a TM7SF2 inhibitor) against the neurodegenerative disease of the patient.
[3-2] A method, using, as an index, a concentration of sterol (for example, FF-MAS, T-MAS, 14-dehydrozymosterol, or 14-dehydrozymostenol) constituting a cholesterol synthesis pathway in a biological sample of a patient with a disease associated with a disorder of a neuromuscular junction, for determining or predicting efficacy of an FF-MAS metabolism inhibitor (for example, a sterol C14-reductase inhibitor or a TM7SF2 inhibitor) against the disease associated with a disorder of a neuromuscular junction of the patient.
[3-3] A method, using, as an index, an expression level of TM7SF2 in a biological sample of a patient with a neurodegenerative disease, for determining or predicting efficacy of an FF-MAS metabolism inhibitor (for example, a sterol C14-reductase inhibitor or a TM7SF2 inhibitor) against the neurodegenerative disease of the patient.
[3-4] A method, using, as an index, an expression level of LBR or an expression level of TM7SF2 (preferably an expression level of TM7SF2) in a biological sample of a patient with a disease associated with a disorder of a neuromuscular junction, for determining or predicting efficacy of an FF-MAS metabolism inhibitor (for example, a sterol C14-reductase inhibitor or a TM7SF2 inhibitor) against the disease associated with a disorder of a neuromuscular junction of the patient.
[4-1] A method, using, as an index, a concentration of sterol constituting a cholesterol synthesis pathway in a biological sample of a patient with a neurodegenerative disease, for determining or predicting onset, severity, or prognosis of the neurodegenerative disease of the patient.
[4-2] A method, using, as an index, a concentration of sterol constituting a cholesterol synthesis pathway in a biological sample of a patient with a disease associated with a disorder of a neuromuscular junction, for determining or predicting onset, severity, or prognosis of the disease associated with a disorder of a neuromuscular junction of the patient.
[4-3] A method, using, as an index, an expression level of TM7SF2 in a biological sample of a patient with a neurodegenerative disease, for determining or predicting onset, severity, or prognosis of the neurodegenerative disease of the patient.
[4-4] A method, using, as an index, an expression level of LBR or an expression level of TM7SF2 (preferably an expression level of TM7SF2) in a biological sample of a patient with a disease associated with a disorder of a neuromuscular junction, for determining or predicting onset, severity, or prognosis of the disease associated with a disorder of a neuromuscular junction of the patient.
[5-1] A method for protecting and/or regenerating a neuromuscular junction or a method for protecting and/or regenerating a nervous system cell, the method including administering an effective amount of an FF-MAS metabolism inhibitor (for example, a sterol C14-reductase inhibitor or a TM7SF2 inhibitor) to a mammal.
[5-2] A method for treating and/or preventing a neurodegenerative disease or a myogenic disease, the method including administering an effective amount of an FF-MAS metabolism inhibitor (for example, a sterol C14-reductase inhibitor or a TM7SF2 inhibitor) to a mammal (a patient in need thereof).
[5-3] A method for treating and/or preventing a disease associated with a disorder of a neuromuscular junction, the method including administering an effective amount of an FF-MAS metabolism inhibitor (for example, a sterol C14-reductase inhibitor or a TM7SF2 inhibitor) to a mammal (a patient in need thereof).
[5-4] An FF-MAS metabolism inhibitor (for example, a sterol C14-reductase inhibitor or a TM7SF2 inhibitor) for use in protecting and/or regenerating a neuromuscular junction or protecting and/or regenerating a nervous system cell.
[5-5] An FF-MAS metabolism inhibitor (for example, a sterol C14-reductase inhibitor or a TM7SF2 inhibitor) for use in treating and/or preventing a neurodegenerative disease or a myogenic disease.
[5-6] An FF-MAS metabolism inhibitor (for example, a sterol C14-reductase inhibitor or a TM7SF2 inhibitor) for use in treating and/or preventing a disease associated with a disorder of a neuromuscular junction.
[5-7] A use of an FF-MAS metabolism inhibitor (for example, a sterol C14-reductase inhibitor or a TM7SF2 inhibitor) for manufacturing an agent for protecting and/or regenerating a neuromuscular junction or an agent for protecting and/or regenerating a nervous system cell.
[5-8] A use of an FF-MAS metabolism inhibitor (for example, a sterol C14-reductase inhibitor or a TM7SF2 inhibitor) for manufacturing an agent for treating and/or preventing a neurodegenerative disease or a myogenic disease.
[5-9] A use of an FF-MAS metabolism inhibitor (for example, a sterol C14-reductase inhibitor or a TM7SF2 inhibitor) for manufacturing an agent for treating and/or preventing a disease associated with a disorder of a neuromuscular junction.

### ADVANTAGEOUS EFFECTS OF INVENTION

The drug of the present invention (e.g., an agent containing an FF-MAS metabolism inhibitor) can be used for protecting and/or regenerating a neuromuscular junction and/or a nervous system cell.

### BRIEF DESCRIPTION OF DRAWINGS

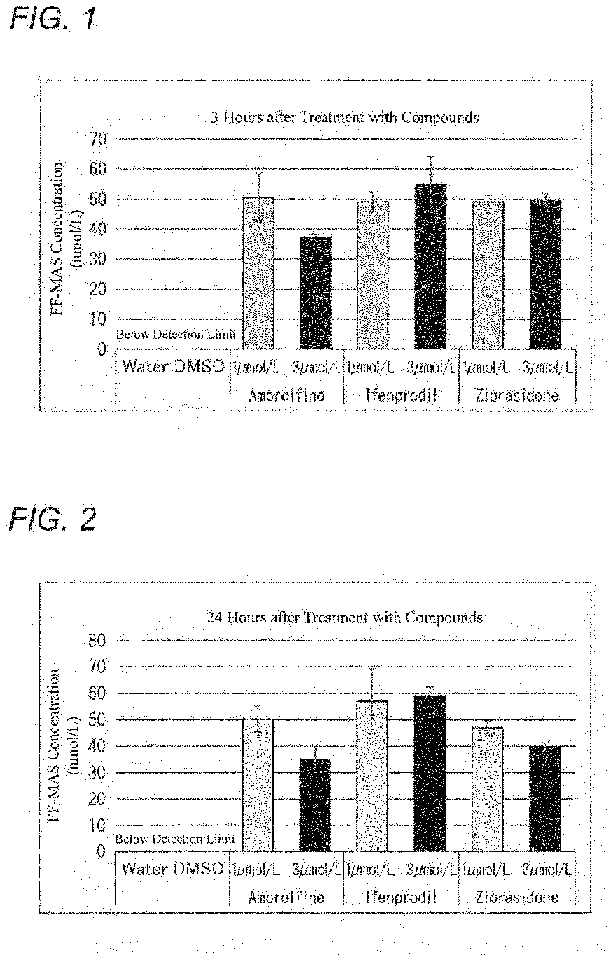

Fig. 1 shows the FF-MAS concentration in C2C12 cells at 3 hours after treatment when mouse myoblast cell line C2C12 cells into which human mutant SOD1 (G93A) was introduced were treated with TM7SF2 inhibitors (the results are shown as mean ± standard deviation for each group).
Fig. 2 shows the FF-MAS concentration in C2C12 cells at 24 hours after treatment when mouse myoblast cell line C2C12 cells into which human mutant SOD1 (G93A) was introduced were treated with TM7SF2 inhibitors (the results are shown as mean ± standard deviation for each group).

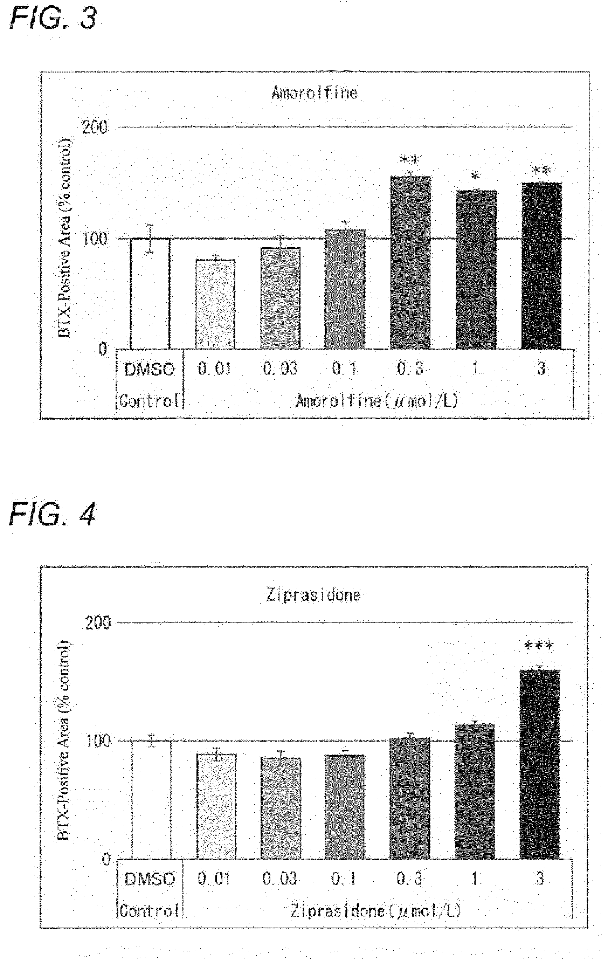

Fig. 3 shows the α-bungarotoxin (BTX)-positive area on day 11 after treatment when a co-culture system of mouse myoblast cell line C2C12 cells into which human mutant SOD1 (G93A) was introduced and mouse primary cultured motor neurons was treated with amorolfine (the results are shown as mean ± standard error for each group, with the mean of the control group being 100%. Control group; N = 6 wells, amorolfine treatment group; each N = 3 wells. *; p < 0.05, **; p < 0.01 (Dunnett's test, versus control)).
Fig. 4 shows the α-bungarotoxin (BTX)-positive area on day 11 after treatment when a co-culture system of mouse myoblast cell line C2C12 cells into which human mutant SOD1 (G93A) was introduced and mouse primary cultured motor neurons was treated with ziprasidone (the results are shown as mean ± standard error for each group, with the mean of the control group being 100%. Control group; N = 6 wells, ziprasidone treatment group; each N = 3 wells. ***; p < 0.001 (Dunnett's test, versus control)).

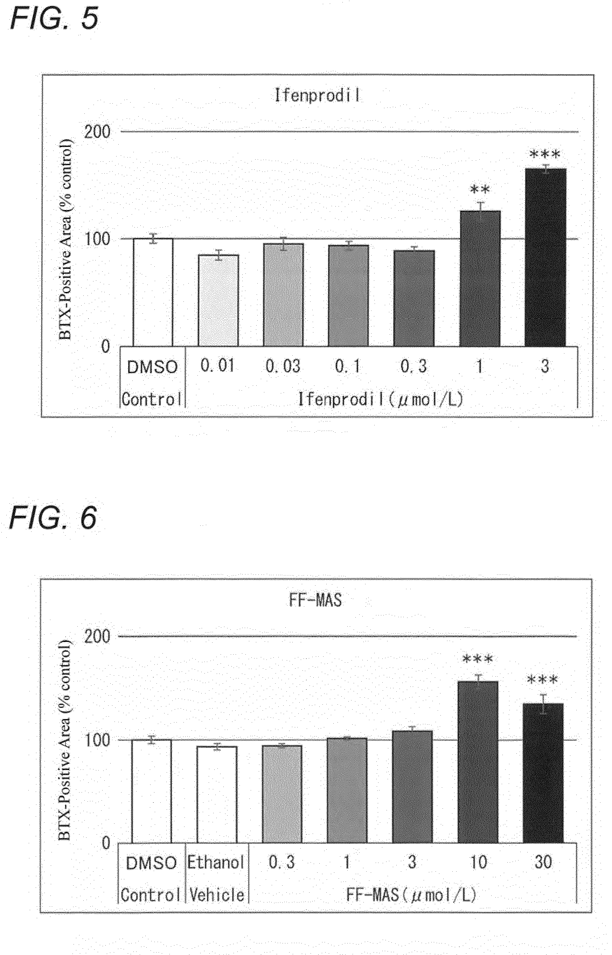

Fig. 5 shows the α-bungarotoxin (BTX)-positive area on day 11 after treatment when a co-culture system of mouse myoblast cell line C2C12 cells into which human mutant SOD1 (G93A) was introduced and mouse primary cultured motor neurons was treated with ifenprodil (the results are shown as mean ± standard error for each group, with the mean of the control group being 100%. Control group; N = 6 wells, ifenprodil treatment group; each N = 3 wells. ^{∗∗}; p < 0.01, ^{∗∗∗}; p < 0.001 (Dunnett's test, versus control)).
Fig. 6 shows the α-bungarotoxin (BTX)-positive area on day 11 after treatment when a co-culture system of mouse myoblast cell line C2C12 cells into which human mutant SOD1 (G93A) was introduced and mouse primary cultured motor neurons was treated with FF-MAS (the results are shown as mean ± standard error of 6 wells in each group, with the mean of the control group being 100%. ***; p < 0.001 (Dunnett's test, versus control)).

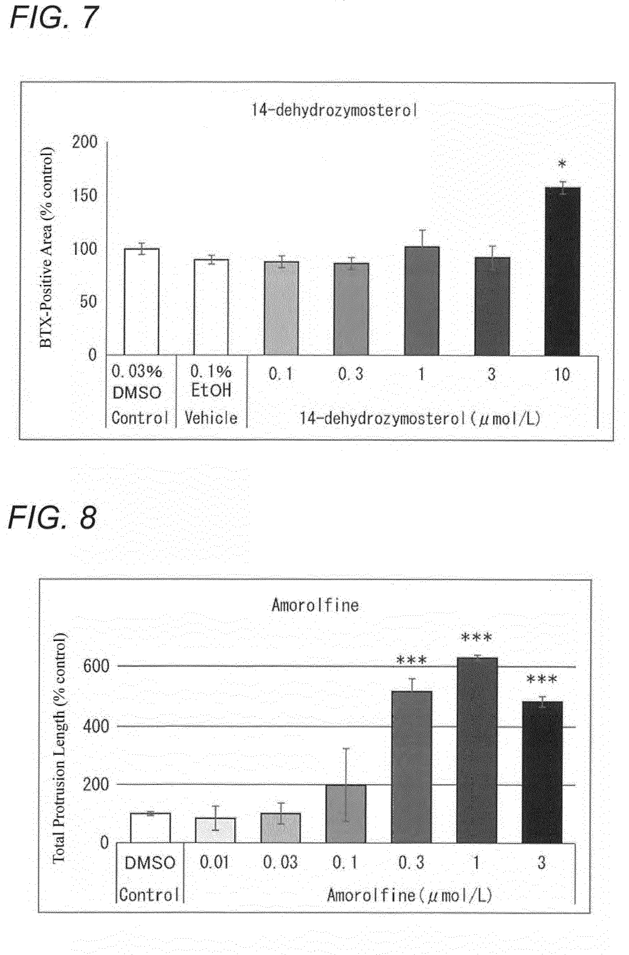

Fig. 7 shows the α-bungarotoxin (BTX)-positive area on day 11 after treatment when a co-culture system of mouse myoblast cell line C2C12 cells into which human mutant SOD1 (G93A) was introduced and mouse primary cultured motor neurons was treated with 14-dehydrozymosterol (the results are shown as mean ± standard error for each group, with the mean of the control group being 100%. Control group; N = 6 wells, EtOH and 14-dehydrozymosterol group; each N = 3 wells. *; p < 0.05, (Dunnett's test, versus control)).
Fig. 8 shows the total protrusion length (sum of neurite lengths) on day 4 after treatment when mouse primary cultured motor neurons were treated with amorolfine (the results are shown as mean ± standard error for each group, with the mean of the control group being 100%. Control group; N = 6 wells, amorolfine treatment group; each N = 3 wells. ***; p < 0.001 (Dunnett's test, versus control)).

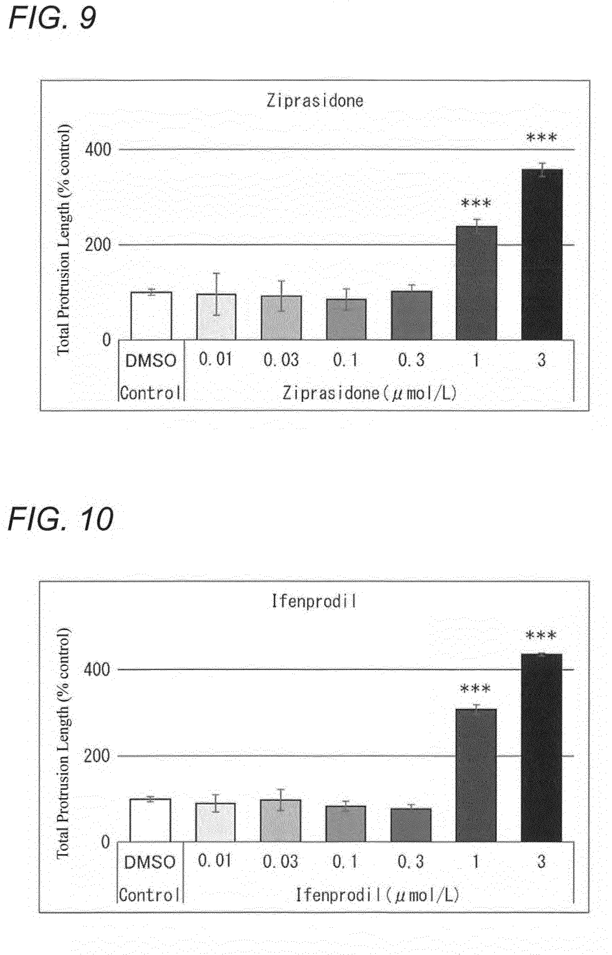

Fig. 9 shows the total protrusion length (sum of neurite lengths) on day 4 after treatment when mouse primary cultured motor neurons were treated with ziprasidone (the results are shown as mean ± standard error for each group, with the mean of the control group being 100%. Control group; N = 6 wells, ziprasidone treatment group; each N = 3 wells. ***; p < 0.001 (Dunnett's test, versus control)).
Fig. 10 shows the total protrusion length (sum of neurite lengths) on day 4 after treatment when mouse primary cultured motor neurons were treated with ifenprodil (the results are shown as mean ± standard error for each group, with the mean of the control group being 100%. Control group; N = 6 wells, ifenprodil treatment group; each N = 3 wells. ^{∗∗∗}; p < 0.001 (Dunnett's test, versus control)).

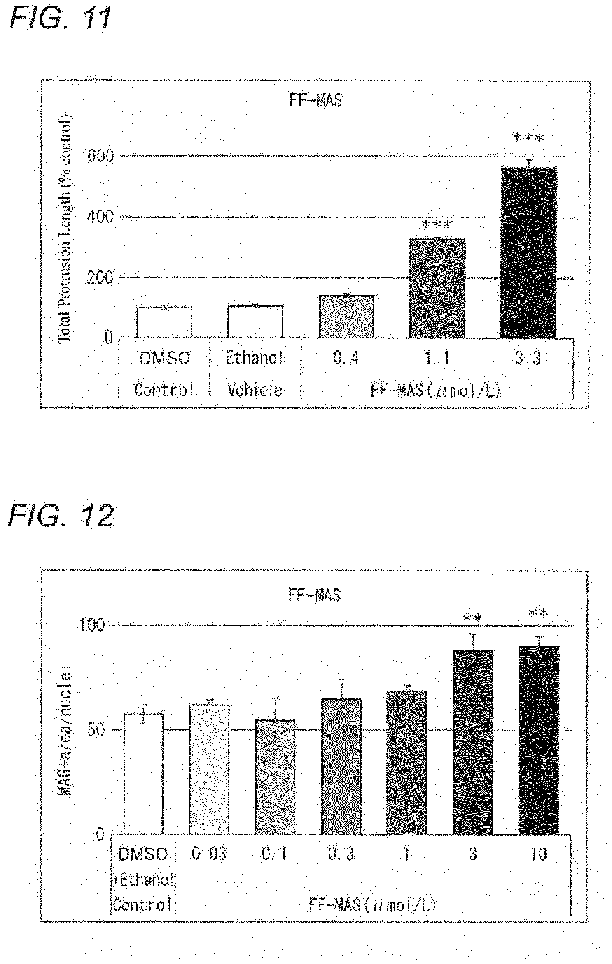

Fig. 11 shows the total protrusion length (sum of neurite lengths) on day 4 after treatment when mouse primary cultured motor neurons were treated with FF-MAS (the results are shown as mean ± standard error of 6 wells in each group, with the mean of the control group being 100%. ***; p < 0.001 (Dunnett's test, versus control)).
Fig. 12 shows the ratio of the MAG-positive area to the number of cell nuclei (MAG+ area/nuclei) calculated from the MAG-positive area and the number of cell nuclei on day 3 after treatment when rat primary Schwann cells were treated with FF-MAS (the results are shown as mean ± standard error for each group. Control group; N = 6 wells, FF-MAS treatment group; each N = 3 wells. **; p < 0.01 (Dunnett's test, versus control)).

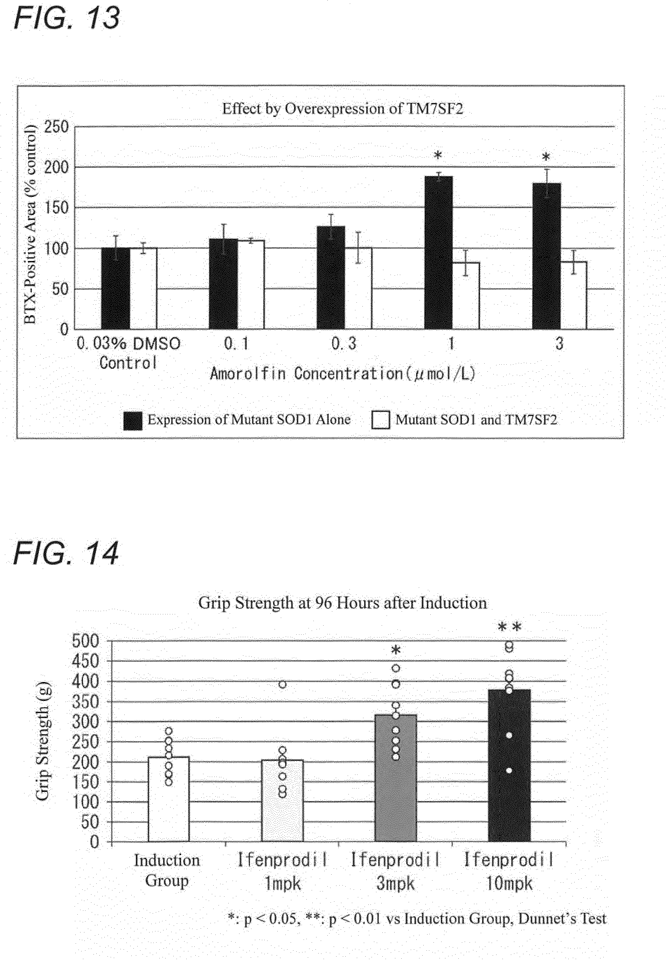

Fig. 13 shows the α-bungarotoxin (BTX)-positive area on day 11 after treatment when a co-culture system of mouse myoblast cell line C2C12 cells in which human mutant SOD1 (G93A) alone or both human mutant SOD1 (G93A) and a human TM7SF2 gene were overexpressed and mouse primary cultured motor neurons was treated with amorolfine (the results are shown as mean ± standard error for each group, with the mean of the control group being 100%. Control group; N = 6 wells, amorolfine treatment group; each N = 3 wells. *; p < 0.05 (Dunnett's test, versus control)).
Fig. 14 shows the results of the efficacy of a TM7SF2 inhibitor (ifenprodil) in a myasthenia gravis model rat (the results represent grip strengths at 96 hours after induction by an anti-acetylcholine receptor antibody, and individual values and mean are shown for each group. *; p < 0.05, **; p < 0.01, (Dunnett's test, versus control)). In the figure, mpk represents mg/kg.

### DESCRIPTION OF EMBODIMENTS

Follicular fluid meiosis activating sterol (FF-MAS) is a kind of sterol constituting a cholesterol synthesis pathway, and refers to 4,4-dimethyl-5alpha-cholesta-8,14,24-trien-3beta-ol and a compound represented by the following structure.

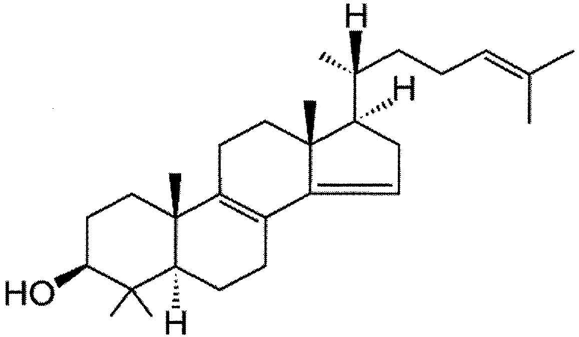

14-dehydrozymosterol is a kind of sterol constituting a cholesterol synthesis pathway, and refers to Salpha-cholesta-8,14,24-trien-3beta-ol biosynthesized by demethylation of a dimethyl group at the 4-position from FF-MAS and a compound represented by the following structure.

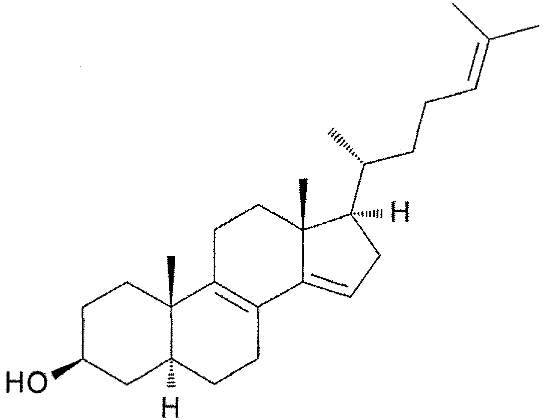

Sterol C14-reductase (it may also be referred to as Δ14 sterol reductase) is an enzyme having a function of metabolizing FF-MAS into testis meiosis-activating sterol (T-MAS) (4,4-dimethyl-5 alpha-cholesta-8,24-diene-3 beta-ol), and examples of a certain aspect include transmembrane 7 superfamily member 2 (TM7SF2) and lamin B receptor (LBR).

In the present invention, an "FF-MAS metabolism inhibitor" refers to a substance having a function of directly or indirectly (preferably, directly) inhibiting the metabolism of FF-MAS in vivo. As the FF-MAS metabolism inhibitor, a substance having an action of increasing the intracellular or extracellular FF-MAS and/or 14-dehydrozymosterol concentration by its inhibitory action is preferable, and examples of a certain aspect include a sterol C14-reductase inhibitor, a TM7SF2 inhibitor, and an LBR inhibitor.

In the present invention, the "sterol C14-reductase inhibitor" refers to a substance that directly or indirectly (preferably, directly) inhibits the function of sterol C14-reductase in vivo. As the sterol C14-reductase inhibitor, a substance having an action of increasing the intracellular or extracellular FF-MAS concentration and/or 14-dehydrozymosterol by its inhibitory action is preferable, and examples of a certain aspect include a TM7SF2 inhibitor and an LBR inhibitor. The sterol C14-reductase inhibitor is preferably a TM7SF2 inhibitor and/or an LBR inhibitor, and more preferably a TM7SF2 inhibitor. Examples of a certain aspect of the sterol C14-reductase inhibitor include amorolfine, ifenprodil, and ziprasidone. The "sterol C14-reductase inhibitor" in the present invention includes not only those that have been found so far but also those that will be found in the future.

In the present invention, the "TM7SF2 metabolism inhibitor" refers to a substance that directly or indirectly (preferably, directly) inhibits the function of TM7SF2 in vivo. As the TM7SF2 inhibitor, a substance having an action of increasing the intracellular or extracellular FF-MAS concentration by its inhibitory action is preferable, and its inhibitory activity is, for example, preferably 10 µmol/L or less, more preferably 1 µmol/L or less, and still more preferably 0.1 µmol/L or less in the IC50 value. Examples of a certain aspect of the TM7SF2 inhibitor include amorolfine, ifenprodil, and ziprasidone. The TM7SF2 inhibitor in the present invention includes not only those that have been found so far but also those that will be found in the future. For example, the TM7SF2 inhibitor can be screened using the screening method (evaluation system) described in Example 8 mentioned below. Examples of the TM7SF2 inhibitor include a compound that exhibits an IC50 value of 10 µmol/L or less (preferably 1 µmol/L or less, and more preferably 0.1 µmol/L or less) in the evaluation system described in Example 8.

In a certain aspect, the TM7SF2 inhibitor is a selective TM7SF2 inhibitor that selectively inhibits TM7SF2.

The selective TM7SF2 inhibitor is, for example, a compound having selectivity for CYP51 and/or EBP (preferably CYP51, preferably EBP, and more preferably CYP51 and EBP). In a certain aspect, the selective TM7SF2 inhibitor is a compound that exhibits 10-fold or more, 20-fold or more, 50-fold or more, or 100-fold or more selectivity for CYP51 and/or EBP. In one embodiment, selectivity can be determined by performing affinity selection mass spectrometry (ASMS) on each enzyme or comparing IC50 values in a binding test. The inhibitory activity (IC50 value) against CYP51 and/or EBP can be determined, for example, by adjusting the evaluation system described in Example 8 mentioned below for CYP51 or EBP, or by using a known method, for example, the method described in Non Patent Literature 4.

In the present invention, the "LBR metabolism inhibitor" refers to a substance that directly or indirectly (preferably, directly) inhibits the function of LBR in vivo. As the LBR inhibitor, a substance having an action of increasing the intracellular or extracellular FF-MAS concentration by its inhibitory action is preferable, and its inhibitory activity is, for example, preferably 10 µmol/L or less, more preferably 1 µmol/L or less, and still more preferably 0.1 µmol/L or less in the IC50 value. Examples of a certain aspect of the LBR inhibitor include amorolfine, ifenprodil, and ziprasidone.

It is described in Non Patent Literature 4, Patent Literature 1, and the like that amorolfine, ifenprodil, ziprasidone, and the like have LBR or TM7SF2 inhibitory activity.

The LBR inhibitor in the present invention includes not only those that have been found so far but also those that will be found in the future. For example, the LBR inhibitor can be screened using the evaluation system described in Example 8 mentioned below. Examples of the LBR inhibitor include a compound that exhibits an IC50 value of 10 µmol/L or less (preferably 1 µmol/L or less, and more preferably 0.1 µmol/L or less) in the evaluation system described in Example 8.

In a certain aspect, the LBR inhibitor is a selective LBR inhibitor that selectively inhibits LBR.

The selective LBR inhibitor is, for example, a compound having selectivity for CYP51 and/or EBP. In a certain aspect, the selective LBR inhibitor is a compound that exhibits 10-fold or more, 20-fold or more, 50-fold or more, or 100-fold or more selectivity for CYP51 and/or EBP (preferably CYP51, preferably EBP, and more preferably CYP51 and EBP). In one embodiment, selectivity can be determined by performing ASMS on each enzyme or comparing IC50 values in a binding test.

In a certain aspect, the selective TM7SF2 inhibitor is a compound having selectivity also for LBR. In a certain aspect, the selective TM7SF2 inhibitor is a compound also having inhibitory activity on LBR (a selective TM7SF2 and LBR dual inhibitor).

In a certain aspect, the selective LBR inhibitor is a compound having selectivity also for TM7SF2. In a certain aspect, the selective LBR inhibitor is a compound also having inhibitory activity on TM7SF2 (a selective TM7SF2 and LBR dual inhibitor).

In a certain aspect, the sterol C14-reductase inhibitor is a substance that selectively inhibits sterol C14-reductase. In a certain aspect, the selective sterol C14-reductase inhibitor is a selective TM7SF2 inhibitor, a selective LBR inhibitor, or a selective TM7SF2 and LBR dual inhibitor. The selective TM7SF2 and LBR dual inhibitor is a substance that selectively inhibits TM7SF2 and LBR, and is, for example, a compound having selectivity for CYP51 and/or EBP (preferably CYP51, and more preferably CYP51 and EBP).

In the present invention, the sterol C14-reductase inhibitor may be a combination of any two or more thereof. Specific examples thereof include a combination of a TM7SF2 inhibitor and an LBR inhibitor. In addition, the selective sterol C14-reductase inhibitor may be a combination of any two or more thereof. Specific examples thereof include a combination of a selective TM7SF2 inhibitor and a selective LBR inhibitor.

In the present invention, examples of the FF-MAS metabolism inhibitor, the sterol C14-reductase inhibitor, the TM7SF2 inhibitor, or the LBR inhibitor include, but are not limited to, a low molecular weight compound (for example, amorolfine, ifenprodil, and ziprasidone), a polypeptide (for example, an antibody (preferably an antagonistic antibody) and an antibody fragment), and a nucleic acid (for example, an antisense and siRNA).

### [Pharmaceutical Use]

In a certain aspect, the FF-MAS metabolism inhibitor (for example, a sterol C14-reductase inhibitor or a TM7SF2 inhibitor) in the present invention is useful for protecting and/or regenerating a neuromuscular junction or protecting and/or regenerating a nervous system cell.

In the present invention, "protection of a neuromuscular junction" refers to an action of suppressing a disorder (including functional and morphological disorders and hypoplasia) of a neuromuscular junction.

In the present invention, "regeneration of a neuromuscular junction" refers to an action of regenerating a disordered neuromuscular junction.

In the present invention, "protection and/or regeneration of a neuromuscular junction" can be evaluated by image analysis of a neuromuscular junction formed by co-culturing a nerve cell and a muscle cell after staining an acetylcholinesterase or acetylcholine receptor that is a marker of the neuromuscular junction.

In the present invention, "protection of a nervous system cell" refers to an action of protecting a nervous system cell (for example, a nerve cell and a Schwann cell) from damage and/or degeneration thereof.

In the present invention, "regeneration of a nervous system cell" refers to an action of regenerating a damaged and/or degenerated nervous system cell (for example, a nerve cell and a Schwann cell).

In the present invention, a "neurite outgrowth action" refers to an action of growing the length from the cell body to the neurite terminal of a nerve cell, and can be evaluated, for example, by staining the nucleus and the neurite of the nerve cell and performing image analysis.

In the present invention, a "Schwann cell differentiation-promoting action" refers to an action of promoting a change in state from an undifferentiated Schwann cell to a differentiated Schwann cell that forms a myelin sheath or an action of suppressing a change in state from a differentiated Schwann cell to an undifferentiated Schwann cell that occurs in nerve injury or the like, and can be evaluated, for example, by staining a protein such as myelin associated glycoprotein (MAG) involved in myelin formation and performing image analysis.

In the present invention, "treatment" means, for example, curing or ameliorating a certain disease or a symptom thereof, and "prevention" means preventing or delaying for a certain period of time the onset of a certain disease or a symptom. Treatment and/or prevention includes inhibiting the progression or worsening of a symptom to halt the progression of a pathological condition or preventing recurrence or reducing the likelihood of recurrence of a disease or a symptom.

The FF-MAS metabolism inhibitor (for example, a sterol C14-reductase inhibitor or a TM7SF2 inhibitor) according to the present invention is useful for treating and/or preventing a disease associated with a disorder of a neuromuscular junction (for example, a neurodegenerative disease and a myogenic disease).

Therefore, a certain aspect of the present invention is an agent for treating and/or preventing a disease associated with a disorder of a neuromuscular junction, the agent containing an FF-MAS metabolism inhibitor (for example, a sterol C14-reductase inhibitor or a TM7SF2 inhibitor).

A certain aspect of the neurodegenerative disease is, for example, facial nerve palsy, peripheral neuropathy (for example, diabetic peripheral neuropathy or peripheral neuropathy associated with administration of an anticancer agent), spinal stenosis, glaucoma, age-related macular degeneration, Alzheimer's disease, dementia with Lewy bodies, progressive supranuclear palsy, corticobasal degeneration, Parkinson's disease, multiple system atrophy, amyotrophic lateral sclerosis, bulbospinal muscular atrophy, primary lateral sclerosis, spinal muscular atrophy, spinocerebellar degeneration, frontotemporal lobar degeneration, Huntington's disease, Charcot-Marie-Tooth disease, or traumatic neurodegenerative disease, and is preferably peripheral neuropathy, Alzheimer's disease, Parkinson's disease, or amyotrophic lateral sclerosis. Another aspect of the neurodegenerative disease is a neurodegenerative disease associated with a disorder of a neuromuscular junction or a nervous system cell (for example, a nerve cell (for example, a peripheral nerve cell) and a Schwann cell).

A certain aspect of the myogenic disease is, for example, myasthenia gravis, muscular dystrophy, sarcopenia, or myopathy.

Therefore, a certain aspect of the present invention is an agent for protecting and/or regenerating a neuromuscular junction, the agent containing an FF-MAS metabolism inhibitor (for example, a sterol C14-reductase inhibitor or a TM7SF2 inhibitor).

Another aspect of the present invention is an agent for protecting and/or regenerating a nervous system cell, the agent containing an FF-MAS metabolism inhibitor (for example, a sterol C14-reductase inhibitor or a TM7SF2 inhibitor).

Another aspect of the present invention is an agent for protecting and/or regenerating a nervous system cell mediated by a neurite outgrowth action, the agent containing an FF-MAS metabolism inhibitor (for example, a sterol C14-reductase inhibitor or a TM7SF2 inhibitor).

Another aspect of the present invention is an agent for protecting and/or regenerating a nervous system cell mediated by a Schwann cell differentiation-promoting action, the agent containing an FF-MAS metabolism inhibitor (for example, a sterol C14-reductase inhibitor or a TM7SF2 inhibitor).

Another aspect of the present invention is a neurite outgrowth agent containing an FF-MAS metabolism inhibitor (for example, a sterol C14-reductase inhibitor or a TM7SF2 inhibitor).

Another aspect of the present invention is a Schwann cell differentiation-promoting agent containing an FF-MAS metabolism inhibitor (for example, a sterol C14-reductase inhibitor or a TM7SF2 inhibitor).

Another aspect of the present invention is an agent for treating and/or preventing a neurodegenerative disease (for example, peripheral neuropathy, Alzheimer's disease, Parkinson's disease, or amyotrophic lateral sclerosis), the agent containing an FF-MAS metabolism inhibitor (for example, a sterol C14-reductase inhibitor or a TM7SF2 inhibitor).

Another aspect of the present invention is an agent for treating and/or preventing a neurodegenerative disease (for example, peripheral neuropathy, Alzheimer's disease, Parkinson's disease, or amyotrophic lateral sclerosis) mediated by an action of protecting and/or regenerating a neuromuscular junction, the agent containing an FF-MAS metabolism inhibitor (for example, a sterol C14-reductase inhibitor or a TM7SF2 inhibitor).

Another aspect of the present invention is an agent for treating and/or preventing a neurodegenerative disease (for example, peripheral neuropathy, Alzheimer's disease, Parkinson's disease, or amyotrophic lateral sclerosis) mediated by a neurite outgrowth action, the agent containing an FF-MAS metabolism inhibitor (for example, a sterol C14-reductase inhibitor or a TM7SF2 inhibitor).

Another aspect of the present invention is an agent for treating and/or preventing a neurodegenerative disease (for example, peripheral neuropathy, Alzheimer's disease, Parkinson's disease, or amyotrophic lateral sclerosis) mediated by a Schwann cell differentiation-promoting action, the agent containing an FF-MAS metabolism inhibitor (for example, a sterol C14-reductase inhibitor or a TM7SF2 inhibitor).

Another aspect of the present invention is an agent for treating and/or preventing a neurodegenerative disease associated with a disorder of a neuromuscular junction (for example, peripheral neuropathy, Alzheimer's disease, Parkinson's disease, or amyotrophic lateral sclerosis), the agent containing an FF-MAS metabolism inhibitor (for example, a sterol C14-reductase inhibitor or a TM7SF2 inhibitor).

Another aspect of the present invention is an agent for treating and/or preventing a neurodegenerative disease (preferably associated with a disorder of a neuromuscular junction) (for example, peripheral neuropathy, Alzheimer's disease, Parkinson's disease, or amyotrophic lateral sclerosis) for protecting and/or regenerating a neuromuscular junction, the agent containing an FF-MAS metabolism inhibitor (for example, a sterol C14-reductase inhibitor or a TM7SF2 inhibitor).

Another aspect of the present invention is an agent for treating and/or preventing a neurodegenerative disease associated with a disorder of a nervous system cell (for example, a neurite or a Schwann cell) (for example, peripheral neuropathy, Alzheimer's disease, Parkinson's disease, or amyotrophic lateral sclerosis), the agent containing an FF-MAS metabolism inhibitor (for example, a sterol C14-reductase inhibitor or a TM7SF2 inhibitor).

Another aspect of the present invention is an agent for treating and/or preventing a neurodegenerative disease (for example, peripheral neuropathy, Alzheimer's disease, Parkinson's disease, or amyotrophic lateral sclerosis) for protecting and/or regenerating a neuromuscular junction and/or protecting and/or regenerating a nervous system cell (for neurite outgrowth and/or Schwann cell differentiation promotion), the agent containing an FF-MAS metabolism inhibitor (for example, a sterol C14-reductase inhibitor or a TM7SF2 inhibitor).

Another aspect of the present invention is an agent for treating and/or preventing a myogenic disease (for example, myasthenia gravis, muscular dystrophy, sarcopenia, or myopathy) mediated by an action of protecting and/or regenerating a neuromuscular junction, the agent containing an FF-MAS metabolism inhibitor (for example, a sterol C14-reductase inhibitor or a TM7SF2 inhibitor).

Another aspect of the present invention is an agent for treating and/or preventing a myogenic disease associated with a disorder of a neuromuscular junction (for example, myasthenia gravis, muscular dystrophy, sarcopenia, or myopathy), the agent containing an FF-MAS metabolism inhibitor (for example, a sterol C14-reductase inhibitor or a TM7SF2 inhibitor).

Another aspect of the present invention is an agent for treating and/or preventing a myogenic disease (preferably associated with a disorder of a neuromuscular junction) (for example, myasthenia gravis, muscular dystrophy, sarcopenia, or myopathy) for protecting and/or regenerating a neuromuscular junction, the agent containing an FF-MAS metabolism inhibitor (for example, a sterol C14-reductase inhibitor or a TM7SF2 inhibitor).

Another aspect of the present invention is an agent for protecting and/or regenerating a neuromuscular junction or an agent for protecting and/or regenerating a nervous system cell, the agent containing FF-MAS or 14-dehydrozymosterol or a derivative thereof.

Another aspect of the present invention is an agent for protecting and/or regenerating a neuromuscular junction or an agent for protecting and/or regenerating a nervous system cell, the agent containing an agent having an action of increasing an FF-MAS and/or 14-dehydrozymosterol concentration (for example, a sterol C14-reductase inhibitor or a TM7SF2 inhibitor).

In addition, sterol constituting a cholesterol synthesis pathway such as FF-MAS and a metabolic enzyme thereof (for example, TM7SF2, LBR, EBP, or DHCR7) are deeply related to a neurodegenerative disease (for example, peripheral neuropathy, Alzheimer's disease, Parkinson's disease, or amyotrophic lateral sclerosis) and a disease associated with a disorder of a neuromuscular junction, and thus can be used as a therapeutic and/or diagnostic biomarker for these diseases. Examples of the sterol constituting a cholesterol synthesis pathway include lanosterol, dihydro lanosterol, FF-MAS, T-MAS, dihydro FF-MAS, 14-dehydrozymosterol, 14-dehydrozymostenol, zymosterol, dehydrolathosterol, zymostenol, lathosterol, 7-DHC, 7-DHD, desmosterol, cholesterol, cholecalciferol, or 25-hydroxycholecalciferol2, and preferably FF-MAS, T-MAS, 14-dehydrozymosterol, or 14-dehydrozymostenol.

Therefore, another aspect of the present invention is a method, using, as an index, a concentration of sterol constituting a cholesterol synthesis pathway in a biological sample of a patient with a neurodegenerative disease or a patient with a disease associated with a disorder of a neuromuscular junction, for determining or predicting efficacy of a therapeutic agent (for example, an FF-MAS metabolism inhibitor, a sterol C14-reductase inhibitor, or a TM7SF2 inhibitor) against the disease of the patient.

Another aspect is a method, using, as an index, an expression level of TM7SF2 or an expression level of LBR (preferably an expression level of TM7SF2) in a biological sample of a patient with a neurodegenerative disease or a patient with a disease associated with a disorder of a neuromuscular junction, for determining or predicting efficacy of a therapeutic agent (for example, an FF-MAS metabolism inhibitor, a sterol C14-reductase inhibitor, or a TM7SF2 inhibitor) against the disease of the patient.

In another aspect of the present invention, the concentration of each sterol constituting a cholesterol synthesis pathway, the ratio of sterol, the expression level of TM7SF2, and/or the expression level of LBR is a biomarker (the present biomarker) that predicts (suggests) the efficacy of an FF-MAS metabolism inhibitor.

Another aspect of the present invention is a pharmaceutical composition for a neurodegenerative disease containing an FF-MAS metabolism inhibitor, wherein the FF-MAS metabolism inhibitor is administered to a patient with a neurodegenerative disease against which the FF-MAS metabolism inhibitor is determined or predicted to be effective by the above method.

Another aspect of the present invention is a pharmaceutical composition for a disease associated with a disorder of a neuromuscular junction containing an FF-MAS metabolism inhibitor, wherein the FF-MAS metabolism inhibitor is administered to a patient with a disease associated with a disorder of a neuromuscular junction against which the FF-MAS metabolism inhibitor is determined or predicted to be effective by the above method.

A predetermined threshold value corresponding to each biomarker mentioned above can be appropriately set. The predetermined threshold value can be set based on, for example, data on the present biomarker in a biological sample (e.g., tissue or blood) collected from a patient with a neurodegenerative disease or a patient with a disease associated with a disorder of a neuromuscular junction. For example, the predetermined threshold value may be set as follows. First, biological samples are collected from a plurality of patients with a neurodegenerative disease or patients with a disease associated with a disorder of a neuromuscular junction who have not received an FF-MAS metabolism inhibitor. After collection of the biological samples, an FF-MAS metabolism inhibitor is administered to patients with a neurodegenerative disease or patients with a disease associated with a disorder of a neuromuscular junction, and the patients are monitored. The response to the FF-MAS metabolism inhibitor is confirmed based on a known evaluation index. Then, the biological samples are classified into samples of patients who responded to the FF-MAS metabolism inhibitor and samples of patients who did not respond to the FF-MAS metabolism inhibitor. For each sample, the present biomarker is measured to obtain a measured value. Then, from the obtained measured value, a value that can distinguish between patients who responded to the FF-MAS metabolism inhibitor and patients who did not respond to the FF-MAS metabolism inhibitor is obtained, and the value is set as the predetermined threshold value. When setting the predetermined threshold value, it is preferable to consider the sensitivity, specificity, positive predictive value, and negative predictive value of determination.

Still another aspect is a method, using, as an index, a concentration of sterol constituting a cholesterol synthesis pathway in a biological sample of a patient with a neurodegenerative disease or a patient with a disease associated with a disorder of a neuromuscular junction, for determining or predicting onset, severity, or prognosis of the disease of the patient.

Still another aspect is a method, using, as an index, an expression level of TM7SF2 or an expression level of LBR (preferably an expression level of TM7SF2) in a biological sample of a patient with a neurodegenerative disease or a patient with a disease associated with a disorder of a neuromuscular junction, for determining or predicting onset, severity, or prognosis of the disease of the patient.

### Preparations

The FF-MAS metabolism inhibitor or the like used in the present invention is usually administered systemically or locally in an oral or parenteral form. Examples of the oral preparation include oral liquid preparations (for example, elixirs, syrups, pharmaceutically acceptable waters, suspensions, and emulsions) and oral solid preparations (for example, tablets (including sublingual tablets and orally disintegrating tablets), pills, capsules (including hard capsules, soft capsules, gelatin capsules, and microcapsules), powders, granules, and troches). Examples of the parenteral preparation include liquid preparations (for example, injections (e.g., intravitreal injections, subcutaneous injections, intravenous injections, intramuscular injections, intraperitoneal injections, and drip infusions), eye drops (for example, aqueous eye drops (e.g., aqueous ophthalmic solutions, aqueous suspension ophthalmic solutions, viscous ophthalmic solutions, and solubilized ophthalmic solutions), non-aqueous eye drops (e.g., non-aqueous ophthalmic solutions, non-aqueous suspension ophthalmic solutions))), external preparations (for example, ointments (e.g., eye ointments)), and ear drops. These preparations may be controlled-release agents such as rapid-release preparations and sustained-release preparations. These preparations can be produced by a known method, for example, the method described in the Japanese Pharmacopoeia.

The oral liquid preparation as the oral preparation is produced, for example, by dissolving, suspending, or emulsifying an active ingredient in a commonly used diluent (for example, purified water, ethanol, or a mixture thereof). Furthermore, this liquid preparation may contain a wetting agent, a suspending agent, an emulsifying agent, a sweetening agent, a flavoring agent, an aromatic agent, a preservative, a buffering agent, and the like.

The oral solid preparation as the oral preparation is prepared by, for example, mixing an active ingredient with an excipient (for example, lactose, mannitol, glucose, microcrystalline cellulose, and starch), a binder (for example, hydroxypropyl cellulose, polyvinyl pyrrolidone, and magnesium aluminate metasilicate), a disintegrant (for example, calcium cellulose glycolate), a lubricant (for example, magnesium stearate), a stabilizer, a solubilizing agent (for example, glutamic acid and aspartic acid), or the like according to a conventional method. If necessary, the oral solid preparation may be coated with a coating agent (for example, white sugar, gelatin, hydroxypropyl cellulose, and hydroxypropyl methylcellulose phthalate), or may be coated with two or more layers.

The external preparation as the parenteral preparation is produced by a known method or a commonly used formulation. For example, the ointment is produced by triturating or melting an active ingredient in a base. The ointment base is selected from known or commonly used ones. For example, one selected from higher fatty acids or higher fatty acid esters (for example, adipic acid, myristic acid, palmitic acid, stearic acid, oleic acid, adipic acid ester, myristic acid ester, palmitic acid ester, stearic acid ester, and oleic acid ester), waxes (for example, beeswax, spermaceti wax, and ceresin), surfactants (for example, polyoxyethylene alkyl ether phosphoric acid ester), higher alcohols (for example, cetanol, stearyl alcohol, and cetostearyl alcohol), silicon oils (for example, dimethylpolysiloxane), hydrocarbons (for example, hydrophilic petrolatum, white petrolatum, purified lanolin, and liquid paraffin), glycols (for example, ethylene glycol, diethylene glycol, propylene glycol, polyethylene glycol, and macrogol), vegetable oils (for example, castor oil, olive oil, sesame oil, and turpentine oil), animal oils (for example, mink oil, egg yolk oil, squalane, and squalene), water, absorption promoters, and anti-rash agents, or a mixture of two or more thereof is used. Furthermore, a humectant, a preservative, a stabilizer, an antioxidant, an aromatizing agent, and the like may be contained.

The injection as the parenteral preparation includes a solution, a suspension, an emulsion, and a solid injection that is used after dissolved or suspended in a solvent before use. The injection is used, for example, by dissolving, suspending, or emulsifying an active ingredient in a solvent. As the solvent, for example, distilled water for injection, physiological saline, vegetable oil, and alcohols such as propylene glycol, polyethylene glycol, and ethanol, and a combination thereof are used. Furthermore, the injection may contain a stabilizer, a solubilizing agent (for example, glutamic acid, aspartic acid, and polysorbate 80 (registered trademark)), a suspending agent, an emulsifying agent, a soothing agent, a buffering agent, a preservative, and the like. These are sterilized in the final process or produced by aseptic manipulation. In addition, it is also possible to produce a sterile solid preparation, for example, a freeze-dried product, and dissolve the solid preparation in sterilized or sterile distilled water for injection or another solvent before use.

In order to use the compound of the present invention or a combination agent of the compound of the present invention and another drug for the above purpose, the compound of the present invention or the combination agent of the compound of the present invention and another drug is usually administered systemically or locally in an oral or parenteral form. The dose varies depending on age, body weight, symptom, therapeutic effect, administration method, treatment time, and the like, but is usually orally administered once to several times a day in the range of 1 ng to 1,000 mg per administration per adult, or parenterally administered once to several times a day in the range of 0.1 ng to 10 mg per administration per adult, or continuously administered intravenously in the range of 1 hour to 24 hours per day. Of course, as mentioned above, since the dose varies depending on various conditions, a dose smaller than the above dose may be sufficient, or administration beyond the range may be necessary.

### Combination or Compounding Agent

Furthermore, the FF-MAS metabolism inhibitor or the like used in the present invention may be used in combination with another drug.

In the present invention, the dosage form when the FF-MAS metabolism inhibitor or the like used in combination with another drug (combination) may be a form of a compounding agent in which both components are combined in one preparation, or a dosage form as a separate preparation. The combination can complement the prophylactic, symptom progression-suppressive, recurrence-suppressive, and/or therapeutic effect of another drug, or can maintain or reduce the dose or the frequency of administration. When the FF-MAS metabolism inhibitor or the like used in the present invention and another drug are separately administered, they may be simultaneously administered for a certain period of time, and then only the FF-MAS metabolism inhibitor or the like used in the present invention or only another drug may be administered. In addition, the FF-MAS metabolism inhibitor or the like used in the present invention according to the present invention may be administered first, and another drug may be administered after the completion of the administration, or another drug may be administered first, and the FF-MAS metabolism inhibitor or the like used in the present invention may be administered after the completion of the administration, and each administration method may be the same or different. It is also possible to provide a kit of a preparation containing the FF-MAS metabolism inhibitor or the like used in the present invention and a preparation containing another drug. Here, the dose of another drug can be appropriately selected on the basis of a clinically used dose. In addition, any two or more of other drugs may be administered in combination at an appropriate ratio. In addition, the other drugs include not only those that have been found so far but also those that will be found in the future.

For example, the FF-MAS metabolism inhibitor or the like used in the present invention may be used in combination with riluzole, edaravone, mecobalamin, epalrestat, pregabalin, gabapentin, duloxetine, prostaglandin derivatives (e.g., latanoprost), donepezil, rivastigmine, galantamine, memantine, levodopa, dopamine agonists (e.g., pergolide and ropinirole), anticholinergics (e.g., trihexyphenidyl), amantadine hydrochloride, zonisamide, adenosine receptor antagonists (e.g., istradefylline), MAO-B inhibitors (e.g., selegiline hydrochloride), catechol-O-methyltransferase inhibitors (e.g., entacapone), droxidopa, risdiplam, nucleic acid medicines (e.g., nusinersen sodium), and/or gene therapy (e.g., onasemnogene abeparvovec).

The present invention will be described in more detail by the following Examples, but the scope of the present invention is not limited thereto.

### EXAMPLES

### Example 1: FF-MAS and 14-dehydrozymosterol Accumulation Action by Sterol C14-Reductase Inhibitor (TM7SF2 Inhibitor)

### <Experimental Method>

After mouse myoblast cell line C2C12 cells into which human mutant SOD1 (G93A) was introduced were myotube-like differentiated, the cells were treated with sterol C14-reductase inhibitors (TM7SF2 inhibitors) (amorolfine, ziprasidone, and ifenprodil), and the fluctuation of sterol in the cells was evaluated. In other words, in order to differentiate C2C12 cells into myotubes, C2C12 cells that had been cultured using 15% FBS-DMEM (Dulbecco's modified Eagle's medium containing 15% fetal bovine serum and 100 units/mL penicillin-100 µg/mL streptomycin) were cultured for 2 days in a culture flask after the medium was replaced with a low-serum medium (Dulbecco's modified Eagle's medium containing 0.5% FBS, 1% insulin-transferrin-selenium, and 100 units/mL penicillin-100 µg/mL streptomycin). Then, in order to remove proliferating undifferentiated cells, the medium was replaced with a low-serum medium containing 10 µmol/L arabinocytidine (AraC), and the cells were further cultured for 2 days. The myotube-like differentiated C2C12 cells were detached from the culture flask by treatment with 0.25% trypsin-1 mmol/L EDTA, and then the cells were seeded on a 3.5-cm culture dish coated with Matrigel at 2.4 × 10⁵ cells/dish, and cultured for 3 days. After treatment with a sterol C14-reductase inhibitor (TM7SF2 inhibitor) or 0.03% DMSO, the cells were recovered at 3 and 24 hours. After removing the medium and washing with phosphate buffered saline (PBS), the cells were recovered with 200 µL of 90% methanol containing 1 µmol/L d7-cholesterol (internal standard substance), and further recovered again with 200 µL of a 90% methanol solution, so that a total of about 400 µL of a cell recovery solution was obtained. Then, 160 µL of this cell recovery solution was pretreated by a solid-phase extraction method using OASIS PRiME HLB µElution Plate, and a sample eluted with methanol/IPA (1 : 1) was measured by high-performance liquid chromatography/atmospheric pressure chemical ionization-tandem mass spectrometry (LC/APCI-MS/MS), and each concentration in the recovery solution was calculated by an internal standard calibration curve method.

### <Results and Discussion>

The results of FF-MAS are shown in Figs. 1 and 2. In the DMSO treatment group, the FF-MAS concentration in the mouse myoblast cell line C2C12 cells into which human mutant SOD1 (G93A) was introduced was below the detection limit, but when the cells were treated with amorolfine, ziprasidone, and ifenprodil having a sterol C14-reductase inhibitory action (TM7SF2 inhibitory action), an increase in the intracellular FF-MAS concentration was confirmed under any condition. In addition, an increase in the concentration of 14-dehydrozymosterol was confirmed in the same treatment. Therefore, it was confirmed that, by inhibiting sterol C14-reductase (inhibiting TM7SF2), these compounds increase FF-MAS and 14-dehydrozymosterol, which are substrates of this enzyme, in cells.

### Example 2: Neuromuscular Junction Disorder-Ameliorating Action by Sterol C14-Reductase Inhibitor (TM7SF2 Inhibitor), FF-MAS, or 14-dehydrozymosterol

### <Experimental Method>

Using a cell evaluation system for evaluating neuromuscular junction formation by co-culture of mouse myoblast cell line C2C12 cells into which human mutant SOD1 (G93A) was introduced and mouse primary cultured motor neurons, actions of a sterol C14-reductase inhibitor (TM7SF2 inhibitor), FF-MAS, and 14-dehydrozymosterol on neuromuscular junction formation were evaluated. First, in order to differentiate C2C12 cells into myotubes, C2C12 cells that had been cultured using 15% FBS-DMEM were cultured for 2 days in a culture flask after the medium was replaced with a low-serum medium. Then, in order to remove proliferating undifferentiated cells, the medium was replaced with a low-serum medium containing 10 µmol/L AraC, and the cells were further cultured for 2 days. The myotube-like differentiated C2C12 cells were detached from the culture flask by treatment with 0.25% trypsin-1 mmol/L EDTA, and then the cells were seeded on a 96-well imaging plate coated with Matrigel at 1.5 × 10⁴ cells/well, and cultured in a low-serum medium containing AraC for 3 days until the start of co-culture with mouse primary cultured motor neurons. The mouse primary cultured motor neurons were prepared from an embryonic day 13 mouse fetus. A mouse on day 13 of gestation was subjected to cervical dislocation and euthanized, and the abdomen was incised to extract the fetus. The fetus was decapitated, and then the spinal cord was extracted under a stereomicroscope. The spinal cord was treated with HBSS containing 0.25% trypsin at 37°C for 15 minutes, then the supernatant was removed, washing was performed 3 times with HBSS, 2 mL of a dispersion solution (HBSS containing 0.125 mg/mL DNase) was added, and pipetting was repeated with a tapered Pasteur pipette to prepare a cell suspension. The cell suspension was centrifuged (200 g, 5 minutes), 2 mL of a motor neuron medium (Neurobasal medium containing 2% B27 supplement, 1 µmol/L AraC, 5 mmol/L creatine, 10 ng/mL BDNF, 10 ng/mL GDNF, 10 ng/mL CNTF, and 0.125 mmol/L L-glutamine) was added to the sediment, the suspension was resuspended, and the number of cells was measured. The medium of the 96-well imaging plate in which the myotube-like differentiated C2C12 cells had been cultured was all removed, and the mouse primary cultured motor neurons suspended in the motor neuron medium were seeded at 3.0 × 10⁴ cells/100 µL/well to start co-culture. At 4 hours after the start of co-culture, 50 µL of the culture supernatant was removed, and 50 µL of a Matrigel solution diluted 20 times with the motor neuron medium was added. The mixture was allowed to stand at room temperature for 5 minutes, and then cultured in a CO₂ incubator for 1 hour, and 100 µL of the motor neuron medium was added. Furthermore, sterol C14-reductase inhibitors (TM7SF2 inhibitors) (amorolfine, ziprasidone, and ifenprodil) were added at 0.01, 0.03, 0.1, 0.3, 1, and 3 µmol/L, FF-MAS was added at 0.4, 1.1, 3.3, 10, and 30 µmol/L, or 14-dehydrozymosterol was added at 0.1, 0.3, 1, 3, and 10 µmol/L, and the cells were cultured for 11 days until a neuromuscular junction was formed. The neuromuscular junction was evaluated by staining an acetylcholine receptor, which is an index of the neuromuscular junction in the muscle, with α-bungarotoxin. In other words, a 96-well plate for evaluation in which cells were fixed by treating with paraformaldehyde at a final concentration of 1% at room temperature for 20 minutes was washed 3 times with 100 to 150 µL/well of PBS, and PBS containing 2 µg/mL α-bungarotoxin and Alexa Fluor 488 conjugate was added at 50 µL/well. The plate was allowed to stand at room temperature for 1 hour while being shielded from light, and then washed 3 times with 100 to 150 µL/well of PBS. The stained plate was photographed using the Opera Phenix high-throughput high-content imaging system (PerkinElmer). For the photographing of α-bungarotoxin, images of 3 fields per well were acquired using an Alexa Fluor 488 wavelength filter, and the α-bungarotoxin-positive area per field was calculated as the area of the neuromuscular junction by an analysis program. Taking the mean of the area of the neuromuscular junction in the vehicle group (0.03% DMSO or 0.2% ethanol) evaluated for each plate as 100%, the area of the neuromuscular junction in the sterol C14-reductase inhibitor (TM7SF2 inhibitor), FF-MAS, and 14-dehydrozymosterol treatment groups was calculated.

### <Results and Discussion>

The results are shown in Figs. 3 to 7. Amorolfine, ziprasidone and ifenprodil significantly increased the positive area of the neuromuscular junction from 0.3 µmοl/L, 3 µmol/L, and 1 µmol/L, respectively. In addition, FF-MAS and 14-dehydrozymosterol significantly increased the positive area of the neuromuscular junction from 10 µmol/L. Therefore, it was suggested that the sterol C14-reductase inhibitor (TM7SF2 inhibitor) exhibits a formation-promoting action or a protecting action on the neuromuscular junction through an increase in FF-MAS and/or 14-dehydrozymosterol.

When TASIN-1, which is an EBP inhibitor, was added at 0.1 µmol/L and evaluation was performed in the same manner as mentioned above, no significant increase in the positive area of the neuromuscular junction was observed. Furthermore, even when zymosterol was added at 0.05 to 10 µmol/L, no significant increase in the positive area of the neuromuscular junction was observed.

### Example 3: Neurite Outgrowth Action by Sterol C14-Reductase Inhibitor (TM7SF2 Inhibitor) or FF-MAS

### <Experimental Method>

In a mono-culture system of mouse primary cultured motor neurons, the neurite outgrowth action of a sterol C14-reductase inhibitor (TM7SF2 inhibitor) and FF-MAS was evaluated. The mouse primary cultured motor neurons were prepared from an embryonic day 13 mouse fetus. In other words, a mouse on day 13 of gestation was subjected to cervical dislocation and euthanized, and the abdomen was incised to extract the fetus. The fetus was decapitated, and then the spinal cord was extracted under a stereomicroscope. The spinal cord was treated with HBSS containing 0.25% trypsin at 37°C for 15 minutes, then the supernatant was removed, washing was performed 3 times with HBSS, 2 mL of a dispersion solution was added, and pipetting was repeated with a tapered Pasteur pipette to prepare a cell suspension. The cell suspension was centrifuged (200 g, 5 minutes), 2 mL of a motor neuron medium was added to the sediment, the suspension was resuspended, and the number of cells was measured. The mouse primary cultured motor neurons suspended in the motor neuron medium were seeded on a 96-well imaging plate coated with poly-D-lysine at 1.5 × 10⁴ cells/100 µL/well. At 5 hours after the start of culture, a sterol C14-reductase inhibitor (TM7SF2 inhibitor) was added at 0.01, 0.03, 0.1, 0.3, 1, and 3 µmol/L, or FF-MAS was added at 0.4, 1.1, and 3.3 µmol/L, and the cells were cultured for 4 days. The neurite length was evaluated by immunostaining the nuclei and protrusions of nerve cells. In other words, a 96-well plate for evaluation in which cells were fixed by treating with paraformaldehyde at a final concentration of 4% at room temperature for 30 minutes was washed 3 times with 100 to 150 µL/well of PBS, then a blocking solution (PBS containing 5% fetal bovine serum and 0.3% Triton X-100) was added at 100 µL/well, and the plate was allowed to stand at room temperature for 1 hour. As primary antibodies, Anti-Neuron-specific beta-III Tubulin Antibody (R&D Systems, Inc., MAB1195, 1/1,000 dilution) and Anti-NeuN Antibody (Merck Millipore, ABN78, 1/500 dilution) diluted with a blocking solution were added in an amount of 50 µL/well, and the plate was allowed to stand at 4°C overnight. After washing 3 times with 100 to 150 µL/well of PBS, 50 µL/well of Alexa Fluor 647 goat anti-mouse IgG (H+L) (Invitrogen, A-21236, 1/1,000 dilution) and Alexa Fluor 488 goat anti-rabbit IgG (H+L) (Invitrogen, A-11034, 1/1,000 dilution) as secondary antibodies were added, and the plate was allowed to stand at room temperature for 1 hour while being shielded from light, and then washed 3 times with 100 to 150 µL/well of PBS. The stained plate was photographed using the Opera Phenix high-throughput high-content imaging system (PerkinElmer Japan). Images of 5 fields per well were acquired using an Alexa Fluor 647 wavelength filter for photographing neurites and an Alexa Fluor 488 wavelength filter for photographing nuclei of nerve cells, nuclei of nerve cells and neurites per field were analyzed by an analysis program, and the sum of neurite lengths per field was calculated. Taking the mean of the sum of neurite lengths in the vehicle group (0.03% DMSO or 0.2% ethanol) evaluated for each plate as 100%, the sum of neurite lengths in the sterol C14-reductase inhibitor (TM7SF2 inhibitor) and FF-MAS treatment groups was calculated.

### <Results and Discussion>

The results are shown in Figs. 8 to 11. Amorolfine, ziprasidone and ifenprodil significantly increased the sum of neurite lengths from 0.3 µmol/L, 1 µmol/L, and 1 µmol/L, respectively. In addition, FF-MAS significantly increased the sum of neurite lengths from 1.1 µmol/L. Therefore, it was suggested that the sterol C14-reductase inhibitor (TM7SF2 inhibitor) exhibits a neurite outgrowth action or a protecting action through an increase in FF-MAS.

### Example 4: Schwann Cell Differentiation-Promoting Action by Sterol C14-Reductase Inhibitor (TM7SF2 Inhibitor) or FF-MAS

### <Experimental Method>

The Schwann cell differentiation-promoting action of FF-MAS was evaluated in a culture system of rat primary Schwann cells. In other words, neonatal rats aged 0 to 2 days after birth were decapitated, then sterilized by being immersed in ethanol for disinfection, moved to a clean bench, and then washed with a recovery medium (Dulbecco's modified Eagle medium (Sigma Aldrich, product number D5921) without phenol red containing 1 vol% Antibiotic-Antimycotic (Antibiotic-Antimycotic 100×, Thermo Fisher Scientific, Inc., product number 15240062)). Thereafter, the dorsal root ganglion was extracted under a microscope and recovered in a 15 mL tube filled with the recovery medium. The recovered tube was centrifuged at 400 g for 3 minutes at room temperature, and the supernatant was removed. Then, 3 mL of a 0.25% collagenase (Worthington Biochemical Corporation, product number LS004196) solution dissolved in the recovery medium was added to the sediment, and the sediment was broken by pipetting, and then the mixture was incubated in a thermostatic chamber at 37°C for 30 minutes. After incubation, the mixture was centrifuged at 400 g for 3 minutes at room temperature, and the supernatant was removed. To the sediment, 3 mL of a 0.25% trypsin/EDTA solution (FUJIFILM Wako Pure Chemical Corporation, product number 209-16941) and 1 µL of DNaseI (ThermoFisher Scientific, Inc., product number 18047-019) were added, and the sediment was broken by pipetting, and then the mixture was incubated in a thermostatic chamber at 37°C for 30 minutes. After incubation, a culture medium (phenol red-containing DMEM (Sigma Aldrich, product number D6546) containing inactivated 10 vol% fetal bovine serum (Fetal Bovine Serum, qualified, E.U.-approved, South America origin, Thermo Fisher Scientific, Inc., product number 10270106) and 1 vol% Antibiotic-Antimycotic (Thermo Fisher Scientific, Inc.)) was added to inactivate trypsin, the mixture was centrifuged at 400 g for 3 minutes at room temperature, and the supernatant was removed. The culture medium was added to the sediment to prepare a cell suspension, and the cell suspension was passed through a filter (with a diameter of 70 µm). The number of cells in the filtered cell suspension was counted and adjusted to 2.0 × 10⁵ cells/mL, and then the cells were seeded on a 96-well plate coated with poly-D-lysine at 100 µL/well each, and statically cultured in a CO₂ incubator under conditions of 5%CO₂, 95% air, and 37°C. In evaluating the Schwann cell differentiation-promoting action of FF-MAS, the medium of the cell culture plate was removed by aspiration with an aspirator, the medium was replaced with a medium for evaluation (phenol red-containing DMEM (Sigma Aldrich, product number D6546) containing inactivated 1 vol% dialyzed fetal bovine serum (Fetal Bovine Serum, dialyzed, US origin, Thermo Fisher Scientific, Inc., product number 26400044), 1 vol% Antibiotic-Antimycotic (Thermo Fisher Scientific, Inc.), and 100 µmol/L db-cAMP (Sigma-Aldrich, product number D0627)), FF-MAS was added at 0.03, 0.1, 0.3, 1, 3, and 10 µmol/L, and the cells were cultured for 3 days. To the vehicle group, 0.1% DMSO and 0.1% ethanol were added. The Schwann cell differentiation-promoting action was evaluated on the basis of the expression level of myelin-associated glycoprotein (hereinafter referred to as MAG). After removing the medium, 100 µL each of 4% paraformaldehyde (FUJIFILM Wako Pure Chemical Corporation, 163-20145) was added, and the mixture was allowed to stand at room temperature for 20 minutes or more to fix the cells. After removing the supernatant, 100 µL each of a Block Ace solution containing 0.1% Tween-20 was added, and the mixture was allowed to stand at room temperature for 30 minutes or more. After removing the supernatant, 60 µL each of a 5 µg/mL anti-MAG antibody (Millipore, product number MAB1567) solution was added, and the mixture was allowed to stand at room temperature for 60 minutes or more. After washing 3 times with PBS, 70 µL each of a 10 µg/mL Alexa Fluor 488 anti-mouse IgG (Thermo Fisher Scientific, Inc., product number A11029) solution was added, and the mixture was allowed to stand at room temperature for 60 minutes or more. After washing 3 times with PBS, 100 µL each of a 10 µg/mL Hoechst33342 (Invitrogen, product number H-3570) solution was added to stain the nuclei. Thereafter, fluorescence images of 9 fields per well were acquired using the Opera Phenix (PerkinElmer). The MAG-positive area and the number of cell nuclei in the acquired fluorescence images were calculated, the sum of 9 fields/well each was defined as the MAG-positive area and the number of cell nuclei of each well, and the value obtained by dividing the MAG-positive area of each well by the number of cell nuclei (MAG/number of cell nuclei) was calculated. In the above method, by adding a sterol C14-reductase inhibitor (TM7SF2 inhibitor) instead of FF-MAS, the Schwann cell differentiation-promoting action of the sterol C14-reductase inhibitor (TM7SF2 inhibitor) can be evaluated.

### <Results and Discussion>

The results of Schwann cell differentiation-promoting action of FF-MAS are shown in Fig. 12. FF-MAS significantly increased the value of MAG/number of cell nuclei from 3 µmol/L. Therefore, it was suggested that FF-MAS has a Schwann cell differentiation-promoting action.

### Example 5: Effect of Overexpression of TM7SF2 on Neuromuscular Junction Disorder-Ameliorating Action by Sterol C14-Reductase Inhibitor (TM7SF2 Inhibitor)

### <Experimental Method>

Using a cell evaluation system for evaluating neuromuscular junction formation by co-culture of mouse myoblast cell line C2C12 cells in which human mutant SOD1 (G93A) alone or both human mutant SOD1 (G93A) and a human TM7SF2 gene were overexpressed and mouse primary cultured motor neurons, an action of amorolfm, which is a sterol C14-reductase inhibitor (TM7SF2 inhibitor), on neuromuscular junction formation was evaluated. First, in order to differentiate C2C12 cells into myotubes, C2C12 cells that had been cultured using 15% FBS-DMEM were cultured for 2 days in a culture flask after the medium was replaced with a low-serum medium. Then, in order to remove proliferating undifferentiated cells, the medium was replaced with a low-serum medium containing 10 µmol/L AraC, and the cells were further cultured for 2 days. The myotube-like differentiated C2C12 cells were detached from the culture flask by treatment with 0.25% trypsin-1 mmol/L EDTA, and then the cells were seeded on a 96-well imaging plate coated with Matrigel at 1.5 × 10⁴ cells/well, and cultured in a low-serum medium containing AraC for 3 days until the start of co-culture with mouse primary cultured motor neurons. The mouse primary cultured motor neurons were prepared from an embryonic day 13 mouse fetus. A mouse on day 13 of gestation was subjected to cervical dislocation and euthanized, and the abdomen was incised to extract the fetus. The fetus was decapitated, and then the spinal cord was extracted under a stereomicroscope. The spinal cord was treated with HBSS containing 0.25% trypsin at 37°C for 15 minutes, then the supernatant was removed, washing was performed 3 times with HBSS, 2 mL of a dispersion solution (HBSS containing 0.125 mg/mL DNase) was added, and pipetting was repeated with a tapered Pasteur pipette to prepare a cell suspension. The cell suspension was centrifuged (200 g, 5 minutes), 2 mL of a motor neuron medium (Neurobasal medium containing 2% B27 supplement, 1 µmol/L AraC, 5 mmol/L creatine, 10 ng/mL BDNF, 10 ng/mL GDNF, 10 ng/mL CNTF, and 0.125 mmol/L L-glutamine) was added to the sediment, the suspension was resuspended, and the number of cells was measured. The medium of the 96-well imaging plate in which the myotube-like differentiated C2C12 cells had been cultured was all removed, and the mouse primary cultured motor neurons suspended in the motor neuron medium were seeded at 3.0 × 10⁴ cells/100 µL/well to start co-culture. At 4 hours after the start of co-culture, 50 µL of the culture supernatant was removed, and 50 µL of a Matrigel solution diluted 20 times with the motor neuron medium was added. The mixture was allowed to stand at room temperature for 5 minutes, and then cultured in a CO₂ incubator for 1 hour, and 100 µL of the motor neuron medium was added. Furthermore, amorolfine was added at 0.1, 0.3, 1, and 3 µmol/L, and the cells were cultured for 11 days until a neuromuscular junction was formed. The neuromuscular junction was evaluated by staining an acetylcholine receptor, which is an index of the neuromuscular junction in the muscle, with α-bungarotoxin. In other words, a 96-well plate for evaluation in which cells were fixed by treating with paraformaldehyde at a final concentration of 1% at room temperature for 20 minutes was washed 3 times with 100 to 150 µL/well of PBS, and PBS containing 2 µg/mL α-bungarotoxin and Alexa Fluor 488 conjugate was added at 50 µL/well. The plate was allowed to stand at room temperature for 1 hour while being shielded from light, and then washed 3 times with 100 to 150 µL/well of PBS. The stained plate was photographed using the Opera Phenix high-throughput high-content imaging system (PerkinElmer). For the photographing of α-bungarotoxin, images of 3 fields per well were acquired using an Alexa Fluor 488 wavelength filter, and the α-bungarotoxin-positive area per field was calculated as the area of the neuromuscular junction by an analysis program. Taking the mean of the area of the neuromuscular junction in the vehicle group (0.03% DMSO) evaluated for each plate as 100%, the area of the neuromuscular junction in the sterol C14-reductase inhibitor (TM7SF2 inhibitor) treatment group was calculated.

### <Results and Discussion>

The results are shown in Fig. 13. In the combination of C2C12 cells in which human mutant SOD1 (G93A) was overexpressed alone and mouse primary motor neurons, amorolfine significantly increased the positive area of the neuromuscular junction at 1 µmol/L and 3 µmol/L. On the other hand, in the combination of C2C12 cells in which both human mutant SOD1 (G93A) and a human TM7SF2 gene were overexpressed and mouse primary motor neurons, even when the cells were treated with amorolfine in the same concentration range, no increasing fluctuation was found in the positive area of the neuromuscular junction. This result indicates that the increasing action of amorolfine on the positive area of the neuromuscular junction is canceled by overexpression of TM7SF2, and thus it is considered that amorolfine exhibits a formation-promoting action or a protecting action on the neuromuscular junction by inhibiting TM7SF2.

### Example 6: Evaluation of Efficacy of Sterol C14-Reductase Inhibitor (TM7SF2 Inhibitor) on Myasthenia Gravis

### <Experimental Method>

The efficacy of ifenprodil, which is a sterol C14-reductase inhibitor (TM7SF2 inhibitor), in a rat myasthenia gravis model was evaluated. The rat myasthenia gravis model is a model in which a neuromuscular junction (NMJ) is impaired and motor functions such as grip strength are declined by treating a rat with mAb35, which is an antibody against an anti-acetylcholine receptor. First, an anti-acetylcholine receptor antibody mAb35 (Bio X Cell, product number BE0123) as an inducing agent was intraperitoneally administered to a 5-week-old female LEW/CrlCrlj rat at a concentration of 0.29 mg/mL and a volume of 5 mL/kg to induce a pathological condition. Ifenprodil (MedChemExpress, product number HY-12882A) as a test substance was dissolved in water for injection (Otsuka Pharmaceutical Factory, Inc.) containing 2% DMSO so that the concentration is 0.1, 0.3, and 1 mg/mL to prepare an administration solution. Ifenprodil was repeatedly orally administered once a day at doses of 1, 3, and 10 mL/kg from 9 hours after induction by administration of mAb35 to 4 days after induction as the evaluation end date.

As the animals, animals that had no abnormality during a 1-week acclimation period were used. The grip strength was measured on the day before induction (pre-value). At 9 hours after induction, the grip strength was measured again to calculate a rate of decline in grip strength from the pre-value, and only animals that had a certain decline in grip strength by induction were used for evaluation. For quantitative evaluation of the grip strength, a grip strength measuring device Traction Meter (BrainScience idea Co., Ltd.) was used.

### <Results and Discussion>

The results are shown in Fig. 14. In the myasthenia gravis model rat, ifenprodil significantly improved the grip strength at 96 hours after induction from 3 mg/kg. Therefore, the sterol C14-reductase inhibitor (TM7SF2 inhibitor) was shown to be effective against myasthenia gravis.

### Example 7: Evaluation of Efficacy of Sterol C14-Reductase Inhibitor (TM7SF2 Inhibitor) on ALS

Human mutant SOD1 (G93A) transgenic mice are used as model mice reflecting a pathological condition of ALS. Mice of the B6SJL-Tg(SOD^{∗}G93A) 1 Gur/J strain, which is one type of these transgenic mice, start to show declines in grip strength, myogenic potential, motor coordination, and fatigue resistance from around 50 days of age after birth, start to show a decrease in body weight at around 90 days of age after birth, and start to die at around 120 days of age after birth. By monitoring the course of these symptoms reflecting a pathological condition of ALS, the efficacy of a sterol C14-reductase inhibitor (TM7SF2 inhibitor) in ALS can be evaluated.

### Example 8: Method for Screening TM7SF2 Inhibitor or LBR Inhibitor

### <Experimental Method>

The IC50 of an inhibitor is calculated using an enzyme source prepared by expressing human TM7SF2 and LBR in Expi293F cells to form a membrane fraction. A DMSO solution of an inhibitor candidate is dispensed into a 96-well plate using a D300e microdispenser so that the final concentration of the inhibitor candidate is 0.0003, 0.001, 0.003, 0.01, 0.03, 0.1, 0.3, 1, 3, and 10 µmol/L (DMSO has a final concentration of 0.3 v/v%), DMSO is added so that the final concentration is 0.3 v/v%, and this is defined as an inhibitor candidate non-addition group. A substrate solution prepared in a 0.1 mol/L potassium phosphate buffer (pH 7.4) containing 0.1 w/v% BSA so that the final concentration is 3 µmol/L FF-MAS and a protein solution prepared in a 0.1 mol/L potassium phosphate buffer (pH 7.4) containing 0.1 w/v% BSA so that the final concentration is 0.01 mg/mL protein human TM7SF2 or human LBR-expressing membrane fraction are added to the 96-well plate into which the inhibitor candidate was dispensed. After preincubation at 37°C for 5 minutes, an NADPH solution prepared in a 0.1 mol/L potassium phosphate buffer (pH 7.4) containing 0.1 w/v% BSA is added so that the final concentration is 2 mmol/L, and the mixture is allowed to stand at 37°C to start an enzymatic reaction.

After 1 hour from the start of the enzymatic reaction, methanol containing 1 µmol/L d7-cholesterol is added, the mixture is stirred with a pipette to stop the enzymatic reaction, and the plate is sealed with an aluminum seal. The mixture is allowed to stand for 5 minutes, and then centrifuged at 2,300 g for 5 minutes at 4°C. The production amount of T-MAS produced by the enzymatic reaction using FF-MAS as a substrate is measured by LC/MS/MS, and the IC50 value of each inhibitor candidate is calculated from the T-MAS concentration using Excelfit.

## Claims

1. An agent for protecting and/or regenerating a neuromuscular junction, the agent comprising an FF-MAS metabolism inhibitor.

2. The agent according to claim 1, wherein the FF-MAS metabolism inhibitor is a sterol C14-reductase inhibitor.

3. The agent according to claim 2, wherein the sterol C14-reductase inhibitor is a TM7SF2 inhibitor and/or an LBR inhibitor.

4. The agent according to claim 2, wherein the sterol C14-reductase inhibitor is a TM7SF2 inhibitor.

5. The agent according to claim 2, wherein the sterol C14-reductase inhibitor is a selective sterol C14-reductase inhibitor.

6. The agent according to claim 5, wherein the selective sterol C14-reductase inhibitor is a selective TM7SF2 inhibitor.

7. The agent according to any one of claims 1 to 6, for treating and/or preventing a disease associated with a disorder of a neuromuscular junction.

8. The agent according to claim 7, wherein the disease associated with a disorder of a neuromuscular junction is a neurodegenerative disease or a myogenic disease.

9. The agent according to claim 8, wherein the disease associated with a disorder of a neuromuscular junction is the neurodegenerative disease.

10. The agent according to claim 9, wherein the neurodegenerative disease is peripheral neuropathy, Alzheimer's disease, Parkinson's disease, or amyotrophic lateral sclerosis.

11. The agent according to claim 8, wherein the disease associated with a disorder of a neuromuscular junction is the myogenic disease.

12. The agent according to claim 11, wherein the myogenic disease is myasthenia gravis, muscular dystrophy, sarcopenia, or myopathy.

13. An agent for treating and/or preventing a disease associated with a disorder of a neuromuscular junction, the agent comprising an FF-MAS metabolism inhibitor.

14. The agent according to claim 13, for protecting and/or regenerating a neuromuscular junction.

15. The agent according to claim 13 or 14, wherein the disease associated with a disorder of a neuromuscular junction is a neurodegenerative disease or a myogenic disease.

16. The agent according to claim 15, wherein the disease associated with a disorder of a neuromuscular junction is the neurodegenerative disease.

17. The agent according to claim 16, further for protecting and/or regenerating a nervous system cell.

18. The agent according to claim 15, wherein the disease associated with a disorder of a neuromuscular junction is the myogenic disease.

19. A method for protecting and/or regenerating a neuromuscular junction, the method comprising administering an effective amount of an FF-MAS metabolism inhibitor to a mammal.

20. A method for treating and/or preventing a disease associated with a disorder of a neuromuscular junction, the method comprising administering an effective amount of an FF-MAS metabolism inhibitor to a mammal.

21. An FF-MAS metabolism inhibitor for use in protecting and/or regenerating a neuromuscular junction.

22. An FF-MAS metabolism inhibitor for use in treating and/or preventing a disease associated with a disorder of a neuromuscular junction.

23. A use of an FF-MAS metabolism inhibitor for manufacturing an agent for protecting and/or regenerating a neuromuscular junction.

24. A use of an FF-MAS metabolism inhibitor for manufacturing an agent for treating and/or preventing a disease associated with a disorder of a neuromuscular junction.
